# EUROPÄISCHE PATENTANMELDUNG

(11) **EP 0 665 231 A1**
(43) Veröffentlichungstag der Anmeldung: **02.08.1995**
(21) Anmeldenummer: 94890017.0
(22) Anmeldetag: 24.01.1994
(51) Int. Cl.: C07D 491/22, C07D 471/20, A61K 31/435, B01D 11/02

(54) **Verfahren zur Gewinnung von Oxindolalkaloiden**

(71) Anmelder: IMMODAL PHARMAKA GESELLSCHAFT m.b.H., A-6111 Volders (AT)
(72) Erfinder: Keplinger, Dietmar, A-6020 Innsbruck (AT)
(74) Vertreter: Torggler, Paul, Dr.

(57) **Zusammenfassung**

Zur Gewinnung von Oxindolalkaloiden aus einer Oxindolalkaloide enthaltenden Masse wird die Masse mit Kohlendioxid in verdichtetem, insbesondere überkritischem Zustand mittels eines Schleppmittels extrahiert. Der Extrakt wird zusammen mit dem Kohlendioxid von der extrahierten Masse getrennt und anschließend, inbesondere durch Überführung in die Gasphase, das Kohlendioxid vom Extrakt getrennt. Gegebenenfalls wird der Extrakt dann noch von coextrahierten Substanzen gereinigt.

## Beschreibung

Die Erfindung betrifft ein Verfahren zur Gewinnung von Oxindolalkaloiden aus einer Oxindolalkaloide enthaltenden Masse, beispielsweise aus Pflanzenteilen.

Unter einer Oxindolalkaloide enthaltenden Masse werden hierbei nicht nur Pflanzenteile wie Wurzeln oder Wurzelteile, Blätter, Stammteile, Triebe, sondern auch Zellkulturen aus nativen oder modifizierten Zellen, sowie Massen mit synthetisch hergestellten Oxindolalkaloiden verstanden. Beispiele für synthetisch hergestellte Oxindolalkaloide sind in Chem.Pharm.Bull. 23(11)2605-2613(1975), Ban, Seto und Oishi, Total Synthesis of Alkaloids (±)-Phynchophylline and (±)-Isorynchophylline, in Chem.Pharm.Bull. 24(4)736-751(1976), Ban, Taga und Oishi, Total Synthesis of (±)-Formosanine, (±)-Isoformosanine, (±)-Mitraphylline und (±)-Isomitraphylline, oder in Tetrahydron Letters, Vol. 31, pp 4557-4560, 1990, Martin und Mortimore, Total Synthesis of Isopterpodine and Pterpodine beschrieben.

Für die Extraktion von Pflanzenteilen zur Gewinnung der enthaltenen Oxindolalkaloide werden verschiedene Lösungsmittel verwendet. So beschreibt beispielsweise die WO-A 82/01130 die Extraktion mittels Wasser, Ethanol, Chloroform und Ethylacetat. Das Extraktionsvermögen der physiologisch unbedenklichen Substanz Wasser ist eher gering, sodaß nur eine sehr geringe Ausbeute erzielbar ist. Organische Lösungsmittel führen zu wesentlich besseren Ergebnissen, doch führt die rückstandslose Entfernung eines physiologisch nicht verträglichen Lösungsmittels meist zu Problemen, wobei sogar die Oxindolalkaloiden selbst oder deren Eigenschaften verändert werden können.

Erfindungsgemäß wird eine verbesserte Ausbeute und eine erleichterte Extraktionsmittelentfernung dadurch erreicht, daß die Oxindolalkaloide enthaltende Masse mit Kohlendioxid in verdichtetem Zustand unter Verwendung eines Schleppmittels extrahiert wird, daS der Extrakt und das Extraktionsmittel von der extrahierten Masse getrennt werden, und daß das Extraktionsmittel vom Extrakt getrennt wird und gegebenenfalls der vom Extraktionsmittel befreite Extrakt von coextrahierten Substanzen gereinigt wird.

Die Masse wird bevorzugt mit Kohlendioxid in überkritischem Zustand unter Zusatz des Schleppmittels extrahiert, beispielsweise unter einem Druck von 330 bar und bei einer Temperatur zwischen 40°C und 50°C. Bevorzugt wird als Schleppmittel Wasser, gegebenenfalls Ethanol verwendet. Kohlendioxid weist mit dem Schleppmittel ausreichende Lösungsmitteleigenschaften für Oxindolalkaloide auf, kann aus dem Extrakt leicht entfernt werden und stellt ein ungiftiges und umweltschonendes Extraktionsmittel dar. Auch die Entfernung des Schleppmittels ist ohne Probleme möglich. Die getrennte Zugabe des Schleppmittels erübrigt sich, wenn eine Masse, in der Oxindolalkaloide in Salzform vorliegen, vor der Extraktion mit Alkalien in wässriger Lösung alkalisiert wird, die einen pK_{B}-Wert aufweisen, der größer ist als der pK_{B}-Wert der zu extrahierenden Oxindolalkaloide, da durch die vorherige Befeuchtung und Alkalisierung in wässriger Lösung bereits das Schleppmittel in der Masse enthalten ist. Für die Extraktion von Pflanzenteilen, in denen Oxindolalkaloide in Salzform vorliegen, werden diese zuvor bevorzugt befeuchtet und alkalisiert, wobei der pK_{B}-Wert der verwendeten Alkalien größer als der der zu extrahierenden Oxindolalkaloide ist. Der pK_{B}-Wert der Alkalien liegt bevorzugt zwischen 3 und 5.

Für die Reinigung von coextrahierten Substanzen wird der extraktionsmittelfreie Extrakt in einer Säure emulgiert, wobei sich aufgrund der lipophilen Bestandteile eine wässrige Phase abscheidet, in der die Oxindolalkaloide enthalten sind. Diese werden dann durch Zugabe einer Base ausgefällt. Im Gegensatz zu den beiden zuvor beschriebenen Verfahren, die zu einer mit diversen anderen Stoffen vermischten Rohalkaloidmischung geführt haben, wird bei der Extraktion mittels eines verflüssigten Gases in der beschriebenen bevorzugten Ausführung eine praktisch reine Alkaloidmischung erhalten. Der Gewinnung der Monosubstanzen muß daher keine aufwendige Reinigung vorangehen. Speziell die Hochdruckextraktion mit Kohlendioxid ist daher wirtschaftlich und in industriellem Maßstab anwendbar.

Folgende Extraktionsversuche wurden mit zwei verschiedenen, Oxindolalkaloide in Salzform enthaltenden Rohdrogen 1 und 2, jeweils zerkleinertem Wurzelmaterial von Uncaria tomentosa (WILLD). DC, durchgeführt, wobei der Gesamtalkaloidgehalt jeweils 0,375 Gew.% des trockenen Ausgangsmaterials betrug.

### Versuch 1:

3,9 kg der grob zerkleinerten Rohdroge 1 wurden ohne weitere Vorbehandlung in einem Extraktor mit einem Extraktionsmittelaufwand von 11 kg CO₂/kg Droge mittels reinem CO₂ bei 330 bar und 50°C über 6 Stunden extrahiert. Der Versuch führte zu einer Ausbeute von 20 g (0,51% der Einwaage) eines lipophilen, orangefarbenen, bei Raumtemperatur hochviskosen Extraktes. Weiters wurden ca. 50 g H₂O mitextrahiert. Die gesuchten Alkaloide waren im Extrakt und im Wasser nur in Spuren vorhanden.

### Versuch 2:

2,32 kg der in der Stiftmühle pulverisierten Rohdroge 1 wurden ohne weitere Vorbehandlung in einem Extraktor mit einem Extraktionsmittelaufwand von 9 kg CO₂/kg Droge mittels reinem CO₂ bei 330 bar und 50°C über 6 Stunden extrahiert. Der Versuch führte zu einer Ausbeute von 12,5 g (= 0,54% der Einwaage) eines lipophilen, orangefarbenen, bei Raumtemperatur hochviskosen Extraktes, weiters wurden ca. 30 g H₂O mitextrahiert. Die gesuchten Alkaloide waren im Extrakt und im Wasser nur in Spuren enthalten.

### Versuch 3:

3,8 kg grob zerkleinerte Rohdroge 2 wurden mit 450 g 4% Ammoniaklösung gleichmäßig durchfeuchtet und 12 Stunden bei Raumtemperatur stehen gelassen, 4,0 kg der so vorbehandelten Droge wurden in einem Extraktor mit einem Extraktionsmittelaufwand von 11 kg CO₂/kg Droge mittels reinem CO₂ bei 330 bar und 50°C über 6 Stunden extrahiert. Der Versuch führte zu einer Ausbeute von 23,7 g (0,66% der eingesetzten Originaldroge) eines lipophilen orangefarbenen, bei Raumtemperatur hochviskosen Extraktes. Weiters wurden ca. 150 g H₂O mitextrahiert. Die gesuchten Alkaloide waren sowohl im Extrakt, als auch im Wasser in relevanter Menge nachweisbar.

### Versuch 4:

10 kg grob zerkleinerte Rohdroge 2 wurden mit 1,2 l wässrigem Ammoniak (400 g 25% NH₃ und 800 g H₂O dest.) gut durchfeuchtet und über 40 Stunden bei Raumtemperatur stehen gelassen. 6,0 kg des vorbehandelten Drogenmaterials wurden in einem Extraktor mit einem Extraktionsmittelaufwand von 11 kg CO₂/kg Droge mittels reinem CO₂ bei 330 bar und 40°C 4 Stunden lang extrahiert. Der Versuch führte zu einer Ausbeute von 52 g (0,86% der eingesetzten Originaldroge) eines lipophilen, orangefarbenen, bei Raumtemperatur hochviskosen Extraktes sowie 62 g H₂O. Die gesuchten Alkaloide waren sowohl im Extrakt als auch im Wasser in relevanter Menge nachweisbar.

### Versuch 5:

10 kg grob zerkleinerte Rohdroge 2 wurden mit 1,2 l wässrigem Ammoniak (400 g 25% NH₃ und 800 g H₂O dest.) gut durchfeuchtet und über 40 Stunden bei Raumtemperatur stehen gelassen. 5,2 kg des vorbehandelten Drogenmaterials wurden in einem Extraktor mit einem Extraktionsmittelaufwand von 11 kg CO₂/kg Droge mittels reinem CO₂ bei 330 bar und 40°C 4 Stunden lang extrahiert. Im Anschluß wurde die Extraktionsanlage mit Aceton gespült und die Spüllösung zur Untersuchung gesammelt. Der Versuch führte zu einer Ausbeute von 36 g (0,69% der eingesetzten Originaldroge) eines lipophilen, orangefarbenen, bei Raumtemperatur hochviskosen Extraktes sowie 89 g H₂O. Die gesuchten Alkaloide waren im Extrakt, im Wasser und in der Spüllösung in relevanten Mengen nachweisbar.

### Versuch 6:

10 kg grob zerkleinerte Rohdroge 2 wurden in einem Zwangsmischer mit 2 l 10% KOH Lösung (200 g KOH fest) intensiv und gleichmäßig gemischt, 12 Stunden stehengelassen und anschließend 40 Stunden lang in einer Wärmekolonne getrocknet. 5,3 kg des vorbehandelten Drogenmaterials wurden in einem Extraktor mit einem Extraktionsmittelaufwand von 11 kg CO₂/kg Droge mittels reinem CO₂ bei 330 bar und 40°C über 4 Stunden extrahiert. Der Versuch führte zu einer Ausbeute von 11 g (0,21% der eingesetzten Originaldroge) eines wasserfreien, lipophilen, orangefarbenen, bei Raumtemperatur hochviskosen Extraktes und von 2 g H₂O. Die gesuchten Alkaloide waren im Extrakt und im Wasser nur in Spuren vorhanden.

### Versuch 7:

10 kg grob zerkleinerte Rohdroge 2 wurden in einem Zwangsmischer mit 2 l 10% KOH Lösung (200 g KOH fest) intensiv und gleichmäßig gemischt, 12 Stunden stehengelassen und anschließend 40 Stunden lang in einer Wärmekolonne getrocknet. 4,1 kg des vorbehandelten Drogenmaterials wurden in einem Extraktor mit einem Extraktionsmittelaufwand von 11 kg CO₂/kg Droge mittels reinem CO₂ bei 330 bar und 40°C über 4 Stunden extrahiert. Anschließend wurde die Extraktionsanlage mit Aceton gespült und die Spüllösung zur Untersuchung gesammelt. Der Versuch führte zu einer Ausbeute von 13 g (0,32% der eingesetzten Originaldroge) eines wasserfreien lipophilen, orangefarbenen, bei Raumtemperatur hochviskosen Extraktes sowie von 3,5 g H₂O. Die gesuchten Alkaloide waren im Extrakt, im Wasser und in der Spüllösung nur in Spuren vorhanden.

Die aus den Versuchen 3 bis 5 gewonnenen Extrakte wurden jeweils
a) in 3,7%iger Salzsäure und
b) mit 25%iger Essigsäure
in einer Konzentration von 2 g Extrakt/100 ml Säure bei einer Temperatur von 80°C gelöst und 1 Stunde lang bei 80°C mit dem Magnetrührer gerührt. Anschließend erfolgte die Phasentrennung durch Ruhigstellen für 3 Stunden bei 5°C. Die Trennung der lipophilen, hochviskosen und der wässrigen Phase erfolgte durch Filtration. Mit der lipophilen Phase wurde der Extraktionsvorgang wiederholt, anschließend wurden die beiden gewonnenen wässrigen Phasen vereinigt. Die wässrige Phase wurde durch tropfenweise Zugabe von NaOH (2mol/l) auf pH 6,5 eingestellt und 3 Stunden lang bei Raumtemperatur digeriert. Anschließend wurden die gefällten Alkaloide mittels eines leichten Unterdruckes durch einen G3 Glassintertiegel abgesaugt, mit CO₂freiem H₂O nachgewaschen und im Trockenschrank getrocknet.

Zur weiteren Reinigung der aus Versuch 3 nach der Versuchsserie b) gewonnene Alkaloide wurden die kristallinen Substanzen in 5%iger Essigsäure bei Raumtemperatur in einer Konzentration von 500 mg/100 ml gelöst und der Fällungsvorgang wiederholt.

Die Versuchsserien a) und b) erbrachten eine durchschnittliche Ausbeute von 250 mg Alkaloidfraktion je 1 g Extrakt. Dies entspricht einer Menge von 0,165% des für die Versuche 3 bis 5 eingesetzten Rohdroge 2 oder einer Ausbeute von 44% der in Rohdroge 2 enthaltenen Gesamtalkaloide. In der dünnschichtchromatographischen Untersuchung war jeweils ein klar getrennter, mehrere Banden umfassender Spot mit einem Rf-Wert von 0,48/0,47 (Isopteropodin, Pteropodin und Isomitraphyllin) sowie drei schwache Spots mit einem Rf-Wert von 0,42 (Isorhynchophyllin), von 0,39 (Mitraphyllin), und von 0,25 (Rhynchophyllin) detektierbar.

Die Alkaloidfraktion aus Versuchsserie a) hatte laut optischer Beurteilung eine gelblich bräunliche Farbe, was auf eine Verunreinigung der Alkaloide mit einer co-extrahierten Substanz hinwies. Daher wurde die Fraktion zwar organoleptisch und dünnschichtchromatographisch beurteilt, eine HPLC Analyse wurde jedoch nicht durchgeführt.

Hingegen führte die Versuchsserie b) zu einer reinweißen, kristallinen Substanz mit dem für die Alkaloide typischen Geschmack. Aufgrund der zu erwartenden hohen Reinheit der Alkaloidfraktion wurde eine Probe zur genauen qualitativen Festlegung der Inhaltsstoffe einer HPLC Analyse unterzogen. Die Analyse wurde nach der Methode des externen Standards mit Mehrpunkteichung in 5 unabhängigen Läufen durchgeführt. Der Gesamtalkaloidgehalt der Probe betrug 100,000% (σᵣₑₗ= 1,121%), davon 20,741% (σᵣₑₗ=1,374%) Isopteropodin, 76,966% (σᵣₑₗ=0,819%) Pteropodin, 1,515% (σᵣₑₗ=2,608%) Isomitraphyllin, sowie insgesamt 1,142% Mitraphyllin, Rhynchophyllin und Isorhynchophyllin.

## Patentansprüche

1. Verfahren zur Gewinnung von Oxindolalkaloiden aus einer Oxindolalkaloide enthaltenden Masse, beispielsweise aus Pflanzenteilen, dadurch gekennzeichnet, daß die Oxindolalkaloide enthaltende Masse mit Kohlendioxid in verdichtetem Zustand unter Verwendung eines Schleppmittels extrahiert wird, daß der Extrakt und das Extraktionsmittel von der extrahierten Masse getrennt werden, und daß das Extraktionsmittel vom Extrakt getrennt wird und gegebenenfalls der vom Extraktionsmittel befreite Extrakt von coextrahierten Substanzen gereinigt wird.

2. Verfahren nach Anspruch 1, dadurch gekennzeichnet, daß Wasser als Schleppmittel verwendet wird.

3. Verfahren nach Anspruch 1 oder 2, dadurch gekennzeichnet, daß als Extraktionsmittel überkritisches Kohlendioxid mit einem Druck von 330 bar und einer Temperatur zwischen 40°C und 50° C verwendet wird.

4. Verfahren nach einem der Ansprüche 1 bis 3, dadurch gekennzeichnet, daß das Kohlendioxid mit oder ohne Schleppmittel durch Überführung in die Gasphase vom Extrakt getrennt wird.

5. Verfahren nach einem der Ansprüche 1 bis 4, dadurch gekennzeichnet, daß eine Masse, in der Oxindolalkaloide in Salzform vorliegen, vor der Extraktion mit Alkalien in wässriger Lösung alkalisiert wird, die einen pK_{B}-Wert aufweisen, der größer ist als der pK_{B}-Wert der zu extrahierenden Oxindolalkaloide.

6. Verfahren nach Anspruch 5, dadurch gekennzeichnet, daß zur Alkalisierung 5%-ige Ammoniaklösung verwendet wird.

7. Verfahren nach einem der Ansprüche 1 bis 6, dadurch gekennzeichnet, daß für die Reinigung von coextrahierten Substanzen der extraktionsmittelfreie Extrakt in einer Säure emulgiert wird, daß aus der Emulsion die wässrige Phase abgetrennt wird, und daß die Oxindolalkaloide durch Zugabe einer Base aus der wässrigen Phase ausgefällt werden.

8. Verfahren nach Anspruch 7, dadurch gekennzeichnet, daß der extraktionsmittelfreie Extrakt in wässriger, insbesondere 9%-iger bis 11%-iger Essigsäure emulgiert wird.

9. Verfahren nach Anspruch 7, dadurch gekennzeichnet, daß zur Ausfällung der Oxindolalkaloide Natronlauge verwendet wird.
